Europäisches Patentamt

European Patent Office

Office européen des brevets

(19)

(11) Veröffentlichungsnummer: **0 296 335 B1**

# (12) EUROPÄISCHE PATENTSCHRIFT

(45) Veröffentlichungstag der Patentschrift: **14.08.91**

(51) Int. Cl.⁵: **A61F 2/32**

(21) Anmeldenummer: **88106798.7**

(22) Anmeldetag: **28.04.88**

(54) Metallisches Knochenimplantat.

(30) Priorität: **23.06.87 CH 2363/87**

(43) Veröffentlichungstag der Anmeldung:
**28.12.88 Patentblatt 88/52**

(45) Bekanntmachung des Hinweises auf die
Patenterteilung:
**14.08.91 Patentblatt 91/33**

(84) Benannte Vertragsstaaten:
**AT DE FR GB IT**

(56) Entgegenhaltungen:
**EP-A- 0 196 258**
**EP-A- 0 217 034**

(73) Patentinhaber: **GEBRÜDER SULZER AKTIENGE-
SELLSCHAFT**
**Zürcherstrasse 9**
**CH-8401 Winterthur(CH)**

(72) Erfinder: **Frey, Otto**
**Wallrütistrasse 56**
**CH-8400 Winterthur(CH)**
Erfinder: **Koch, Rudolf**
**Oberdorfstrasse 229**
**CH-8267 Berlingen(CH)**

## Beschreibung

Die Erfindung betrifft ein metallisches Knochenimplantat, insbesondere blattartiger Schaft für eine Hüftgelenksprothese, das auf Teilen der Oberfläche mit Kunststoff-Formteilen belegt ist, die aussen eine Gitterstruktur aus einem mindestens zweilagigen Metallgitter für das Einwachsen von Knochengewbe tragen.

Aus der EP-A-0 196 258 und der gattungsbildenden EP-A-0 217 034 sind zur zementfreien Verankerung von Femurkopfprothesen Schäfte bekannt, auf deren metallischem Grundkörper im proximalen Bereich Kunststoff-Formteile aufgeschoben sind. Diese Formteile sind bei der EP-A- 0 217 034 aussen mit einem Drahtgitter für das Einwachsen von Gewebe belegt.

Aufgrund unterschiedlicher Elastizitätseigenschaften entstehen bei wechselnden Belastungen eines Implantates zwischen diesen und dem es umgebenden Knochen Mikrobewegungen, die bei den vorstehend beschriebenen Konstruktionen direkt auf das in das Drahtgitter eingewachsene Knochengewebe übertragen werden. Dies kann zu Schädigungen dieses Gewebes und damit zu Lokkerungen des Implantates führen. Es ist daher Aufgabe der vorliegenden Erfindung, die Uebertragung derartiger Mikrobewegungen auf das Knochengewebe möglichst zu unterbinden.

Erfindungsgemäss wird diese Aufgabe dadurch gelöst, dass die Kunststoff-Formteile durch in Vertiefungen des metallenen Grundkörpers mit seitlichem Spiel eingreifende Abstützungen sowie durch ebenfalls im Grundkörper abgestützte, elastische Vorsprünge im Abstand von der Oberfläche des Grundkörpers elastisch verschiebbar gelagert sind.

Bei der neuen Konstruktion besteht zwischen dem metallenen Grundkörper und dem Kunststoff keine starre Verbindung mehr. Infolge der Elastizität der Vorsprünge und dem Spiel bei der Lagerung der Abstützung "schwimmen" die Formteile gewissermassen auf der Oberfläche des Grundkörpers. Infolgedessen werden Mikrobewegungen des Grundkörpers nicht mehr auf die Formteile und damit auf das Knochengewebe übertragen.

Für den Abstand der Oberflächen von Grundkörper und Formteil sind in erster Linie die Abstützungen "verantwortlich", die beispielsweise als Stempel oder Rippen gestaltet sein können. Die Vorsprünge verhindern ein Verkanten der beiden Teile gegeneinander und führen in gewissem Umfang deren Relativbewegungen; auch sie können beispielsweise als noppenartige Einzelelemente mit beliebigen Querschnitten oder als Rippen ausgeführt sein.

Um ein Einwachsen von Knochengewebe in den Spalt zwischen Grundkörper und Vorteil zu verhindern, ist es vorteilhaft, wenn der Abstand höchstens 1 mm beträgt; denn bekanntlich hat sich gezeigt, dass in einen Spalt oder eine Nut einwachsendes Gewebe nur bis zu einer Tiefe vordringt, die - in erster Näherung ganz grob - der Spaltbreite entspricht. Das Anwachsen von Gewebe am Rand des Spaltes kann minimalisiert werden, wenn die im Abstand voneinander gehaltenen Oberflächen von Grundkörper und Formteil geglättet sind.

Im folgenden wird die Erfindung anhand eines Ausführungsbeispiels im Zusammenhang mit der Zeichnung näher erläutert.

Fig. 1 zeigt schematisch eine Ansicht eines Schaftteiles für eine Femurkopf-Prothese, während

Fig. 2 der Schnitt II-II von Fig. 1 ist.

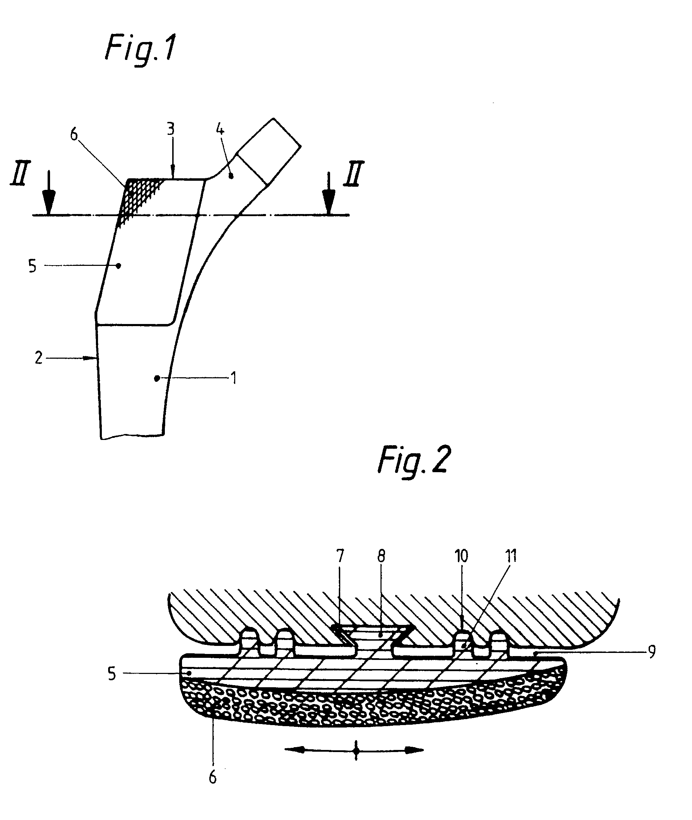

Den Grundkörper des Implantates bildet ein sich nach distal verjüngender Schaft 1 (Fig. 1) für eine Femurkopf-Prothese. Dessen laterale Schmalseite 2 setzt sich an seinem proximalen Ende über eine horizontale Schulter 3 in dem Prothesenhals 4 fort. Der Schaft 1 trägt in seinem proximalen Bereich in bekannter Weise Kunststoff-Formteile 5 aus einem in der Implantat-Technik gebräuchlichen Kunststoff, z.B. aus Polyäthylen medizinischer Qualität. Diese Kunststoff-Formteile 5 sind an ihren äusseren Oberflächen mit einem mehrlagigen Drahtgitter 6 aus Titan oder einer Titanlegierung belegt, in das zur Verankerung des Schaftes 1 Knochengewebe einwachsen soll.

Zur Aufnahme der Formteile 5 hat der Grundkörper bzw. Schaft 1 parallel oder schräg zu seiner Längsachse verlaufende schwalbenschwanzförmige Führungsnuten 7,in die entsprechend ausgebildete Abstützungen oder Rippen 8 der Formteile 5 eingreifen. Erfindungsgemäss ist die aus der Formteiloberfläche hervorspringende "Höhe" der Abstützungen 8 so bemessen, dass zwischen Fcrmteil 5 und Schaft oder Grundkörper 1 ein Spalt 9 vorhanden ist.

Wie Fig. 2 erkennen lässt, sind die Abmessungen der einander angepassten "Schwalbenschwänze" der Nut 7 und der Abstützung 8 so aufeinander abgestimmt, dass ein seitliches Spiel vorhanden ist, das - in Fig. 2 durch einen Doppelpfeil symbolisierte - Relativbewegungen zwischen dem Formteil 5 und dem Grundkörper 1 erlaubt.

Zur Führung dieser Relativbewegungen sind beiderseits der Abstützung 8 in Einbuchtungen 10 des Grundkörpers 1 eingreifende Vorsprünge 11 vorhanden. Diese sind in ihren Querschnitten relativ zu ihrer Höhe - abhängig von dem verwendeten Kunststoff für die Formteile 5 - so bemessen, dass sie elastisch verformt werden, wenn Relativbewegungen zwischen dem Grundkörper 1 und dem Formteil 5 entstehen.

Im vorliegenden Beispiel sind die Einbuchtungen 10 als parallel zur Nut 7 verlaufende Rillen und entsprechend die Vorsprünge 11 als Rippen ausgebildet.

Um das Anwachsen von Gewebe an die einander zugewandten Oberflächen von Grundkörper 1 und Formteil 5 im Spalt 9 zu minimalisieren, können diese Oberflächen geglättet, z.B. poliert sein.

Da bekanntlich die Einwachstiefe von Knochengewebe in Vertiefungen oder Spalte etwa so gross ist wie die Spaltbreite, wird diese möglichst gering gehalten. In der Praxis haben sich Spaltbreiten für den Spalt 9 von 0,1 bis 1 mm bewährt.

Selbstverständlich ist die Erfindung nicht auf die gezeigte Ausführungsform beschränkt; insbesondere die Ausbildung der Vertiefung 7 und der zugehörigen Abstützung 8 sowie der Einbuchtungen 10 und der Vorsprünge 11 kann vielfach variiert werden. So erlauben stempelartige Abstützungen 8 und noppenartige Vorsprünge 11 beispielsweise nicht nur Relativbewegungen in einer Dimension, sondern auch solche in beliebigen Richtungen einer Ebene.

## Patentansprüche

1. Metallisches Knochenimplantat, insbesondere blattartiger Schaft für eine Hüftgelenkprothese, das auf Teilen der Oberfläche seines Grund-Körpers (1) mit Kunststoff-Formteilen (5) belegt ist, die aussen eine Gitterstruktur aus einem mindestens zweilagigen Metallgitter (6) für das Einwachsen von Knochengewebe tragen, dadurch gekennzeichnet, dass die Kunststoff-Formteile (5) durch in Vertiefungen (7) des metallenen Grundkörpers (1) mit seitlichem Spiel eingreifende Abstützungen (8) sowie durch ebenfalls im Grundkörper abgestützte, elastische Vorsprünge (11) im Abstand von der Oberfläche des Grundkörpers (1) elastisch verschiebbar gelagert sind.

2. Knochenimplantat nach Anspruch 1, dadurch gekennzeichnet, dass die im Abstand voneinander gehaltenen Oberflächen von Grundkörper (1) und Formteil (5) geglättet sind.

3. Knochenimplantat nach Anspruch 1, dadurch gekennzeichnet, dass der Abstand höchstens 1 mm beträgt.

## Claims

1. A metal bone implant, more particularly a blade-like stem for a hip joint prosthesis, the implant being covered by plastics mouldings (5) on parts of the surface of its parent member (1), the mouldings (5) having an external mesh structure consisting of ane at least two-layer metal mesh (6) for the invasion of bone tissue, characterised in that, by means of supports (8), which engage in recesses (7) in the metal parent member (1) with lateral clearance, and of resilient projections (11) also supported in the parent member (1), the plastics mouldings (1) are so mounted as to be displaceable resiliently at a distance from the surface of the parent member (1).

2. A bone implant according to claim 1, characterised in that the spaced-apart surfaces of the parent member (1) and moulding (5) are smoothed.

3. A bone implant according to claim 1, characterised in that the gap is at most 1 mm.

## Revendications

1. Implant métallique pour os, notamment tige en forme de lame pour une prothèse d'articulation de la hanche, dont des parties de la surface de son corps de base (1) sont garnies de pièces façonnées (5) en matière plastique qui portent, à l'extérieur, une structure faite d'une grille métallique (6) à au moins deux couches comme substrat de croissance du tissu osseux, caractérisé en ce que les pièces façonnées (5) en matière plastique sont portées, capables d'une translation élastique, à distance de la surface du corps de base (1), par des appuis (8) s'engageant, avec un jeu latéral, dans des creux (7) du corps métallique de base (1) ainsi que par des parties saillantes (11) élastiques, en appui également dans le corps de base.

2. Implant pour os selon la revendication 1, caractérisé en ce que les surfaces du corps de base (1) et de la pièce façonnée (5), maintenues écartées, sont polies.

3. Implant pour os selon la revendication 1, caractérisé en ce que l'écartement est au plus égal à 1 mm.

Fig.1

Fig.2